# EUROPÄISCHE PATENTANMELDUNG

(11) **EP 2 389 911 A1**
(43) Veröffentlichungstag der Anmeldung: **30.11.2011**
(21) Anmeldenummer: 10164008.4
(22) Anmeldetag: 27.05.2010
(51) Int. Cl.: A61F 13/20

(54) **Anordnung mit einem Körper aus absorbierendem Material und einer darin eingesetzten Kapsel**

(71) Anmelder: Marlafin AG, 6332 Hagendorn (CH)
(72) Erfinder: Rolli, Kilian, CH-5400, Baden (CH); Conroy, David, Brooklyn, NY 11231 (US)
(74) Vertreter: AMMANN PATENTANWÄLTE AG BERN

(57) **Zusammenfassung**

Anordnung mit einem Körper (2) aus absorbierendem Material und einer darin eingesetzten Kapsel (3). Die Kapsel (3) weist mindestens eine an ihrem Umfang entlang einer Wendellinie angeordnete Erhebung (6, 11) auf. Zur Herstellung der Anordnung wird die Kapsel (3) durch eine kombinierte rotatorische und translatorische Bewegung in den Körper (2) geschraubt.

## Beschreibung

Die Erfindung betrifft eine Anordnung mit einem Körper aus absorbierendem Material und einer darin eingesetzten Kapsel.

Aus den Dokumenten US5782779,US6183428 und US2007/0260210 sind Tamponanordnungen mit einem im Tamponkörper eingebetteten Vibrationselement bekannt. Solche Tamponanordnungen werden gegen Menstruationsbeschwerden eingesetzt. Was die Einbettung des Vibrationselements in den Tamponkörper anbelangt, kann lediglich dem Dokument US2007/0260210 entnommen werden, dass das Vibrationselement in einer Kapsel enthalten ist, an deren Aussenseite Rückhalteelemente angeordnet sind, um zu verhindern, dass die Kapsel insbesondere beim Zurückziehen der Anordnung aus der Vagina aus dem Tamponkörper herausgerissen wird. Der Tamponkörper ist in diesem Dokument als Hohlkörper beschrieben, in den die Kapsel offenbar hineingesteckt wird.

Ausgehend von diesem Stand der Technik liegt der Erfindung die Aufgabe zu Grunde, eine Anordnung anzugeben, die einfach herstellbar ist und bei welcher die Kapsel im Körper gut verankert ist.

Diese Aufgabe wird erfindungsgemäss dadurch gelöst, dass die Kapsel mindestens eine an ihrem Umfang entlang einer Wendellinie angeordnete Erhebung aufweist.

Diese erfindungsgemässe Lösung hat den Vorteil, dass die mindestens eine Erhebung wie ein Gewinde wirkt, wodurch das Einbringen der Kapsel einfach ist und die Kapsel gut im Körper verankert ist.

Nach einer Ausführungsart ist die Erhebung als wendelförmige Rippe ausgebildet. Dies erlaubt ein einfaches Herstellen der Kapsel.

Nach einer weiteren Ausführungsart weist die Rippe an ihrem Aussenumfang Einschnitte auf. Diese Einschnitte erhöhen den Widerstand gegen ein ungewolltes Zurückdrehen der Kapsel.

Wenn nach einer zusätzlichen Ausführungsart die Einschnitte jeweils zwei in Richtung der Wendellinie orientierte Flanken aufweisen, die unterschiedlich steil ausgebildet sind, lässt sich der Widerstand gegen das Zurückdrehen erhöhen, ohne dass das Einschrauben in gleichem Masse erschwert wird.

Nach einer alternativen Ausführungsart sind eine Vielzahl von Erhebungen entlang einer Wendellinie angeordnet. Dadurch wird die Verankerung der Kapsel im Körper verbessert.

Gemäss einer weiteren Ausführungsart ist vorgesehen, dass die Erhebungen jeweils zwei in Richtung der Wendellinie orientierte Flanken aufweisen, die unterschiedlich steil ausgebildet sind. Dadurch lässt sich die Kapsel verhältnismässig leicht einschrauben, bietet aber einen erheblichen Widerstand gegen das Zurückdrehen.

Nach einer weiteren Ausführungsart ragen von der Mantelfläche der Kapsel und/oder von der Erhebung Fortsätze tangential ab, deren freies Ende in Richtung der Wendellinie entgegengesetzt zur Einschraubrichtung orientiert ist. Diese Fortsätze erfüllen die Funktion von Widerhaken und sorgen für eine zusätzliche Verankerung der Kapsel im Körper.

Nach einer anderen Ausführungsart hat der Körper eine längliche Gestalt. Eine solche Gestalt eignet sich besonders als Tampon für die Monatshygiene der Frau oder für medizinische Anwendungen.

Im Hinblick auf die Gestaltung der Anordnung als Tampon ist es vorteilhaft, wenn nach einer weiteren Ausführungsart das absorbierende Material Fasermaterial ist.

Wenn gemäss einer weiteren Ausführungsart die Kapsel eine längliche Gestalt mit einem Einführungsende und einem rückwärtigen Ende hat, eignet sie sich gut zur Aufnahme in einem Körper mit einer länglichen Gestalt.

Gemäss einer anderen Ausführungsart ist die Kapsel auf mindestens einem Teil ihrer Länge zylindrisch. Diese Gestaltung führt nach dem Einschrauben zu einem guten Kontakt zwischen dem Körper und der Kapsel.

Für das Einschrauben der Kapsel in den Körper ist es vorteilhaft, wenn nach einer weiteren Ausführungsart die Kapsel ein spitz zulaufendes Ende hat.

Entsprechend einer weiteren Ausführungsart nimmt die Höhe der Erhebung bzw. der Erhebungen ausgehend vom Einführungsende der Kapsel zu. Dies erleichtert das Einschrauben der Kapsel in den Körper. Wenn nach einer weiteren Ausführungsart die genannte Höhe gegen das rückwärtige Ende der Kapsel abnimmt, wird ein ungewolltes Trennen der Kapsel vom Körper erschwert oder sogar verunmöglicht, besonders dann, wenn wie weiter oben beschrieben der Körper nach dem Einbringen der Kapsel im Bereich des in Einführungsrichtung rückwärtigen Endes der Kapsel verengt wird.

Eine weitere Ausführungsart sieht vor, dass an der Kapsel ein flexibles Zugmittel befestigt ist. Dieses Zugmittel dient insbesondere zum Zurückziehen einer Anordnung, die in einer Körperöffnung getragen wird.

Nach einer besonderen Ausführungsart ist im Zugmittel mindestens eine Leitung angeordnet. Dabei kann es sich um eine Leitung für elektrischen Strom oder für ein Fluid handeln.

Gemäss einer zusätzlichen Ausführungsart ist in der Kapsel ein Energieverbraucher und/oder ein Sensor angeordnet. Dies eröffnet vielfältige Anwendungen für die Anordnung, insbesondere im Bereich der Medizin.

Schliesslich ist nach einer Ausführungsart der Energieverbraucher ein Vibrationsgenerator. Eine solche Anordnung eignet sich zur Behandlung von Menstruationsbeschwerden.

Die Erfindung betrifft auch ein Verfahren zum Herstellen einer Anordnung der vorangehend beschriebenen Art.

Das Verfahren hat die Aufgabe, eine Kapsel mit wenig Aufwand in einen Körper aus Fasermaterial zu bringen und darin gut zu verankern.

Gelöst wir diese Aufgabe dadurch, dass die Kapsel durch eine kombinierte rotatorische und translatorische Bewegung in den Körper geschraubt wird.

Diese erfindungsgemässe Lösung hat den Vorteil, dass sich die Kapsel durch eine schraubende Bewegung mit verhältnismässig geringem Kraftaufwand in den Körper einbringen lässt.

Nach einer Ausführungsart wird der Körper vor dem Einbringen der Kapsel mit einer Öffnung versehen, in welche die Kapsel eingeschraubt wird. Dadurch erfordert das Einbringen der Kapsel noch weniger Kraftaufwand.

Wenn gemäss einer anderen Ausführungsart der Körper nach dem Einbringen der Kapsel im Bereich des in Einführungsrichtung rückwärtigen Endes der Kapsel verengt wird, erhöht sich die Sicherheit gegen das Herausziehen der Kapsel aus dem Körper. Vorteilhaft erfolgt dabei das Verengen unter Einsatz von Wärme.

Nach einer weiteren Ausführungsart wird die Kapsel im Körper durch einen Faden fixiert. Diese Massnahme dient ebenfalls zur Verbesserung der Verankerung der Kapsel im Körper.

Zur weitere Verbesserung der Verankerung der Kapsel kann eine wie weiter oben beschriebene Kapsel mit Fortsätzen verwendet werden und nach dem Einschrauben um einen Betrag zurück gedreht werden.

Ausführungsbeispiele der Erfindung werden nachstehend unter Bezugnahme auf die angefügten Zeichnungen beispielsweise näher beschrieben. Es zeigt
- Figur 1: eine Seitenansicht einer Anordnung aus einem absorbierenden Körper und einer Kapsel,
- Figur 2: eine Ansicht entsprechend Figur 1 nach dem Verengen des absorbierenden Körpers,
- Figur 3: eine perspektivische Ansicht des absorbierenden Körpers und der Kapsel vor dem Zusammenfügen,
- Figur 4: eine perspektivische Ansicht einer anderen Ausführungsart der Anordnung,
- Figur 5: eine Ansicht von vorne auf eine andere Ausführungsart der Kapsel,
- Figur 6: eine Ansicht von vorne auf eine weitere Ausführungsart der Kapsel und
- Figur 7: eine Ansicht von vorne auf eine weitere Ausführungsart der Kapsel.

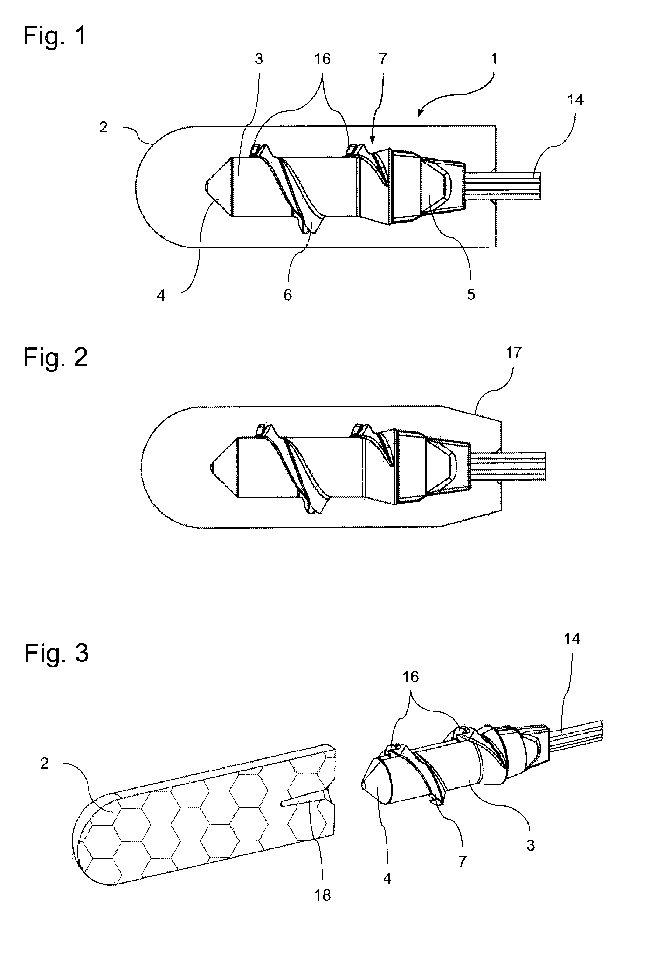

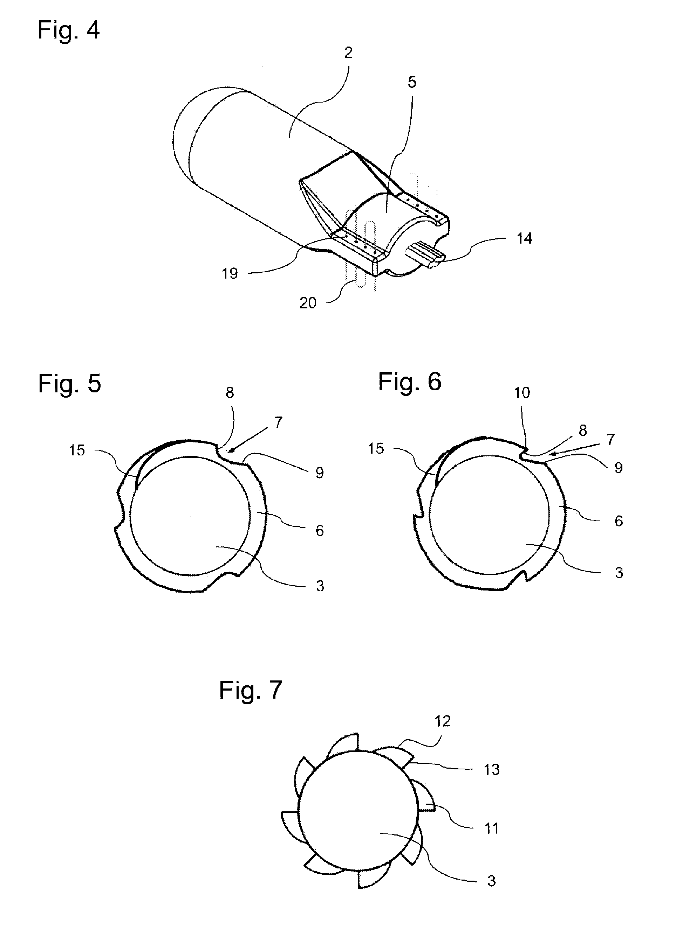

Figur 1 zeigt eine Seitenansicht einer Anordnung 1 aus einem Körper 2 mit einer darin angeordneten Kapsel 3. Der Körper besteht aus einem absorbierenden Material wie beispielsweise Fasermaterial oder Schaumstoff und ist mit einer Umrisslinie dargestellt, damit die darin angeordnete Kapsel 3 gut sichtbar ist. Bei der Anordnung 1 kann es sich beispielsweise um einen vibrierenden Tampon handeln, wie er in den eingangs genannten Dokumenten US5782779,US6183428 und US2007/0260210 beschrieben ist. Die Erfindung ist aber nicht auf vibrierende Tampons beschränkt. Die Kapsel 3 hat ein vorderes Ende 4, das im dargestellten Beispiel spitz zulaufend ist und ein rückwärtiges Ende 5. Am Umfang der Kapsel 3 ist eine wendelförmig umlaufende Rippe 6 angeordnet. Diese Rippe 6 wirkt beim Zusammenfügen der Kapsel 3 mit dem Körper 2 wie ein eingängiges Gewinde. Es könnten auch zwei oder mehr Rippen 6 am Umfang der Kapsel 3 angeordnet sein, die entsprechend wie ein zwei- oder mehrgängiges Gewinde wirken. Am rückwärtigen Ende 5 der Kapsel 3 ist ein flexibles Zugmittel angeordnet, das beispielsweise als Kabel 14 ausgebildet sein kann. Je grösser die Steigung der wendelförmigen Rippe 6 ist, desto grösser ist die Gefahr, dass sich unter einer auf das Kabel 14 wirkenden Zugkraft die Kapsel 3 aus dem Körper 2 zurückdreht. Um dieser Gefahr zu begegnen, sind an der Kapsel 3 Widerhaken 16 angeordnet, deren Spitzen entgegen der Eindrehrichtung orientiert sind. Zusätzlich können in der Rippe 6 Einschnitte 7 vorhanden sein, deren Gestaltung weiter unten im Zusammenhang mit Figur 5 noch näher erläutert wird.

Wie Figur 2 zeigt, kann als eine weitere Sicherung gegen ein ungewolltes Trennen der Kapsel 3 vom Körper 2 nach dem Einschrauben der Kapsel 3 der Körper 2 im Bereich des rückwärtigen Endes 5 der Kapsel 3 verengt werden. Diese Verengung 17 kann durch Aufbringen einer radialen Kraft im genannten Bereich erfolgen. Um diese Verengung dauerhaft zu machen, kann zudem während und/oder nach der Verformung Wärme angewendet werden. Wenn der Körper synthetische Fasern und/oder eine Umhüllung mit synthetischen Fasern enthält, können diese Fasern durch die Wärme angeschmolzen werden und sich so mit benachbarten Fasern verbinden.

Figur 3 zeigt perspektivisch den Körper 2, der hier aufgeschnitten dargestellt ist, und die Kapsel 3 vor dem Zusammenfügen mit dem Körper 2. Letzterer wurde zuvor an seinem rückwärtigen Ende mit einer Öffnung 18 versehen, in welche die Kapsel 3 mit ihrem vorderen Ende 4 eingedrückt und anschliessend eingeschraubt wird. Nach dem vollständigen Einschrauben der Kapsel 3 wird vorzugsweise diese etwas zurückgedreht, damit die an ihrem Umfang angeordneten Widerhaken 16 in das Fasermaterial des Körpers 2 eindringen und so die Kapsel 3 fest im Körper 2 verankern. Anschliessend kann der Körper 2 wie oben beschrieben im Bereich des rückwärtigen Endes 5 der Kapsel 3 verengt werden.

Figur 4 zeigt eine weitere Massnahme zur Sicherung der Kapsel im Körper 2. Dabei sind im rückwärtigen Ende 5 der Kapsel 3 Löcher 19 vorhanden, die es erlauben, den Körper 2 mittels eines Fadens 20 anzunähen. Es ist auch möglich, den Körper 2 mit Hilfe eines Fadens zu verengen, ohne dass der Faden durch Löcher 19 geführt wird. Beispielsweise kann der Faden nach Art einer Tabaksbeutelnaht, wie man sie beispielsweise aus der Chirurgie kennt, durch den Körper 2 geführt werden.

Figur 5 zeigt eine schematische Ansicht einer Kapsel 3 vom vorderen Ende 4 her gesehen. Die Höhe der Rippe 6 steigt von Null bis zu einem Maximum an, wobei dieser Einlaufbereich in der Figur mit 15 bezeichnet ist. Ferner sieht man deutlich die genannten Einschnitte 7, von denen im Beispiel drei pro Umgang der Rippe 6 angeordnet sind. Wie man weiter sieht, haben die Einschnitte 7 jeweils zwei Flanken 8, 9, wobei im dargestellten Beispiel die Flanke 8 steiler ausgebildet ist als die Flanke 9. Dies hat zur Folge, dass beim Einschrauben der Kapsel 3 in den Körper 2 die relativ flache Flanke 9 einen geringeren Widerstand entgegensetzt als die relativ steile Flanke 8 beim Versuch, die Kapsel 3 aus dem Körper 2 herauszuschrauben.

In der Darstellung gemäss Figur 6 ist die Gestaltung der Flanken 8, 9 der Einschnitte 7 so getroffen, dass am äusseren Ende der Flanke 8 eine Spitze 10 entsteht, ähnlich einem Sägezahn. Beim Versuch, die Kapsel 3 aus dem Körper 2 herauszuschrauben, dringen die Spitzen 10 in das Material des Körpers 2 ein und verhindern so eine weitere Rückdrehung.

Figur 7 zeigt ein anderes Beispiel, bei dem nicht eine durchgehende Rippe 6, sondern eine Mehrzahl von Erhebungen 11 am Aussenumfang der Kapsel 3 entlang einer Wendellinie angeordnet sind. Ähnlich wie bei den vorangehend an Hand der Figuren 5 und 6 beschriebenen Beispielen können auch hier die Flanken 12 und 13 der Erhebungen 11 unterschiedliche Neigungen haben.

### Bezugszeichenliste

- 1: Anordnung
- 2: Körper
- 3: Kapsel
- 4: vorderes Ende
- 5: rückwärtiges Ende
- 6: Rippe
- 7: Einschnitt
- 8: Flanke von 7
- 9: Flanke von 7
- 10: Spitze
- 11: Erhebung
- 12: Flanke von 11
- 13: Flanke von 11
- 14: Kabel
- 15: Einlauf
- 16: Widerhaken
- 17: Verengung
- 18: Öffnung
- 19: Loch
- 20: Faden

## Patentansprüche

1. Anordnung mit einem Körper (2) aus absorbierendem Material und einer darin eingesetzten Kapsel (3), **dadurch gekennzeichnet, dass** die Kapsel (3) mindestens eine an ihrem Umfang entlang einer Wendellinie angeordnete Erhebung (6, 11) aufweist.

2. Anordnung nach Anspruch 1, **dadurch gekennzeichnet, dass** die Erhebung als wendelförmige Rippe (6) ausgebildet ist.

3. Anordnung nach Anspruch 2, **dadurch gekennzeichnet, dass** die Rippe (6) an ihrem Aussenumfang Einschnitte (7) aufweist.

4. Anordnung nach Anspruch 3, **dadurch gekennzeichnet, dass** die Einschnitte (7) jeweils zwei in Richtung der Wendellinie orientierte Flanken (8, 9) aufweisen, die unterschiedlich steil ausgebildet sind.

5. Anordnung nach Anspruch 1, **dadurch gekennzeichnet, dass** eine Vielzahl von Erhebungen (11) entlang einer Wendellinie angeordnet sind.

6. Anordnung nach Anspruch 5, **dadurch gekennzeichnet, dass** die Erhebungen (11) jeweils zwei in Richtung der Wendellinie orientierte Flanken (12, 13) aufweisen, die unterschiedlich steil ausgebildet sind.

7. Anordnung nach einem der vorangehenden Ansprüche, **dadurch gekennzeichnet, dass** von der Mantelfläche der Kapsel (3) und/oder von der Erhebung (6, 11) Fortsätze (16) tangential abragen, deren freies Ende in Richtung der Wendellinie entgegengesetzt zur Einschraubrichtung orientiert ist.

8. Anordnung nach einem der vorangehenden Ansprüche, **dadurch gekennzeichnet, dass** der Körper (2) eine längliche Gestalt hat.

9. Anordnung nach einem der vorangehenden Ansprüche, **dadurch gekennzeichnet, dass** das absorbierende Material Fasermaterial ist.

10. Anordnung nach einem der vorangehenden Ansprüche, **dadurch gekennzeichnet, dass** die Kapsel (3) eine längliche Gestalt mit einem Einführungsende (4) und einem rückwärtigen Ende (5) hat.

11. Anordnung nach einem der vorangehenden Ansprüche, **dadurch gekennzeichnet, dass** die Kapsel (3) auf mindestens einem Teil ihrer Länge zylindrisch ist.

12. Anordnung nach einem der Ansprüche 10 bis 11, **dadurch gekennzeichnet, dass** die Kapsel (3) am Einführungsende (4) spitz zulaufend ist.

13. Anordnung nach einem der Ansprüche 10 bis 12, **dadurch gekennzeichnet, dass** die Höhe der Erhebung (6) bzw. der Erhebungen (11) ausgehend vom Einführungsende (4) der Kapsel (3) zunimmt und vorzugsweise gegen das rückwärtige Ende (5) der Kapsel (3) abnimmt.

14. Anordnung nach einem der vorangehenden Ansprüche, **dadurch gekennzeichnet, dass** an der Kapsel (3) ein flexibles Zugmittel (14) befestigt ist.

15. Anordnung nach Anspruch 14, **dadurch gekennzeichnet, dass** im Zugmittel (14) mindestens eine Leitung angeordnet ist.

16. Anordnung nach einem der vorangehenden Ansprüche, **dadurch gekennzeichnet, dass** in der Kapsel (3) ein Energieverbraucher und/oder ein Sensor angeordnet ist.

17. Anordnung nach Anspruch 16, **dadurch gekennzeichnet, dass** der Energieverbraucher ein Vibrationsgenerator ist.

18. Verfahren zum Herstellung einer Anordnung (1) nach einem der vorangehenden Ansprüche, **dadurch gekennzeichnet, dass** die Kapsel (3) durch eine kombinierte rotatorische und translatorische Bewegung in den Körper (2) geschraubt wird.

19. Verfahren nach Anspruch 18, **dadurch gekennzeichnet, dass** der Körper (2) vor dem Einbringen der Kapsel (3) mit einer Öffnung (18) versehen wird, in welche die Kapsel (3) eingeschraubt wird.

20. Verfahren nach einem der Ansprüche 18 bis 19, **dadurch gekennzeichnet, dass** der Körper (2) nach dem Einbringen der Kapsel (3) im Bereich des in Einführungsrichtung rückwärtigen Endes (5) der Kapsel (3) verengt wird.

21. Verfahren nach Anspruch 20, **dadurch gekennzeichnet, dass** das Verengen unter Einsatz von Wärme erfolgt.

22. Verfahren nach einem der Ansprüche 18 bis 20, **dadurch gekennzeichnet, dass** die Kapsel (3) im Körper (2) durch einen Faden (20) fixiert wird.

23. Verfahren nach einem der Ansprüche 18 bis 22 zum Herstellen einer Anordnung nach Anspruch 7, **dadurch gekennzeichnet, dass** die Kapsel nach dem Einschrauben um einen Betrag zurück gedreht wird.
